Europäisches Patentamt

European Patent Office

Office européen des brevets

(11) Publication number: **0 218 188**
**A1**

## (12) EUROPEAN PATENT APPLICATION

(21) Application number: **86113536.6**

(22) Date of filing: **01.10.86**

(51) Int. Cl.4: **G01N 21/82 , G01N 33/546**

(30) Priority: **01.10.85 FI 853783**

(43) Date of publication of application:
**15.04.87 Bulletin 87/16**

(84) Designated Contracting States:
**AT BE CH DE FR GB IT LI NL SE**

(71) Applicant: **Orion Corporation Ltd**
**Saunatontuntie 1**
**SF-02100 Espoo(FI)**

(72) Inventor: **Niskanen, Aimo Juhani**
**Peipontie 20**
**SF-02210 Espoo(FI)**
Inventor: **Kahma, Kauko Ilmari**
**Niittykuja 2 B 42**
**SF-02200 Espoo(FI)**
Inventor: **Vainio, Atte Olavi**
**Joupinmäensyrjä 4 B 44**
**SF-02760 Espoo(FI)**
Inventor: **Lekkala, Jukka Olavi**
**Teekkarinkatu 19 C 43**
**SF-33722 Tampere(FI)**
Inventor: **Joki, Harri Juhani**
**Kortelahdenkatu 22 A 6**
**SF-33230 Tampere(FI)**

(74) Representative: **Vossius & Partner**
**Siebertstrasse 4 P.O. Box 86 07 67**
**D-8000 München 86(DE)**

(54) **An apparatus and method for reading the results of agglutination tests.**

(57) Described is an apparatus which can be used for the carrying out of agglutination tests. The apparatus is used as a base for the reaction in place of the traditional base plate, after which the apparatus is used for estimation of the test result in place of or in addition to the traditional visual observation.

Fig. 1

# AN APPARATUS AND METHOD FOR READING THE RESULTS OF AGGLUTINATION TESTS

The invention consists of an apparatus which can be used for the carrying out of agglutination tests. The apparatus is used as a base for the reaction in place of the traditional base plate, after which the apparatus is used for estimation of the test result in place of or in addition to the traditional visual observation.

Agglutination tests are semiquantitative immunological analysis methods. In agglutination reactions the carrier most frequently used is latex particles. In addition to latex balls, the carrier in agglutination tests may sometimes be particles of an organic material, e.g. substituted or non-substituted polysaccharides, or particles of an inorganic material such as coal, kaolin, bentonite or gold, red blood cells from various animal species, microbial cells such as Staphylococcus or Saccharomyces cells or liposomes. The latex particles, as well as the other particles of organic material, the inorganic particles and the liposomes normally vary in diameter between 0.05 and 3 $\mu$m. In the case of living cells it is of course impossible to influence the size of the particles. The diameter of red blood cells is usually about 7.5 $\mu$m, the size of Saccharomyces-yeast cells is about 5 x 1 m and that of Staphylococcus cells about 1 $\mu$m. The latex particles, as well as the other particles made from organic material and often also the inorganic particles, are present in an aqueous suspension as a milky, homogenous, opalic suspension. The suspension is stabilized by some emulgate, which may be anionic, cationic or a non-polar surface active agent, or the surface charge of the macromolecules themselves. Latex particles are usually prepared from polystyrene, polybutadiene, polymethyl meta-acrylate or polybutyl acrylate. The carriers used in agglutination tests function in the immunological reaction as a kind of amplifier. In such methods a biologically interesting compound, usually one half of a twin-component immunological reaction, i.e. the antibody or the antigen, is attached either chemically, usually covalently, or physically to the surface of a carrier, usually by the technique of adsorption. A few drops of the sample to be investigated, containing an unknown amount of a compound representing one component of an immunological reaction, this component being the substance to be assayed semiquantitatively, are placed usually on two separate, suitably coloured background plates, usually made of non-absorbant material. After this, one or more drops of agglutination reagent are added to the sample drops and negative control reagent may possibly also be added to a separate negative control sample. The samples are mixed and after a suitable time it is inspected whether agglutination has occurred in the sample and comparison may be made with one or more negative samples. Due to the formation of immuno-complex as a result of the possible immunoreaction, bonds may be formed between the carrier particles which are so strong as to destroy the stability of the suspension, thus causing a visible precipitation. The agglutination test can be carried out rapidly; usually the time required for the test is only a few tens of minutes.

The actual carrying out of the agglutination test is quite easy even for individuals with no experience of the performance of this type of analysis. However, the interpretation of the results may be difficult for such individuals. The extent of agglutination may vary considerably, from complete agglutination, when the whole area filled with eg. latex particles becomes granulated and the background remains clear, to partial agglutination when only part of the latex particles become granulated and the background remains milky and opalescent. Strongly positive samples cause complete agglutination in the agglutination test, but a large proportion of positive samples cause only partial agglutination, in which case the interpretation of the results is highly subjective and there is a danger that positive samples may be interpreted as negative.

When interpreting the agglutination caused by weakly positive samples in the agglutination test, the same tester individual may report some samples as positive and some as negative altthough the "different" samples are in fact taken from the same original sample. On the other hand the threshold between positive and negative samples may vary between different individuals, i.e. the same sample may be regarded as definitely positive by one individual but as negative by another.

Attemps have been made to improve the readability and reliability of the results of agglutination tests in many ways. In the case of carriers prepared from latex or other organic material, or from inorganic material, the diameter of the particles has been varied in an attempt to reduce the proportion of questionable agglutination results, or alternatively the optimum colour combination of background and agglutinated particles for the human eye has been investigated in order to improve the practical sensitivity of the visual inspection. In addition, a variety of semi-automatic methods or other methods for the facilitation of the observation of agglutination have been used, eg. microscopical, nephelometric and turbidometric methods and particle counters. The functioning of the particle counters is based on electric charges. The equipment used, however, is usually expensive and difficult to use in practice and the advantages of its use are often disputable.

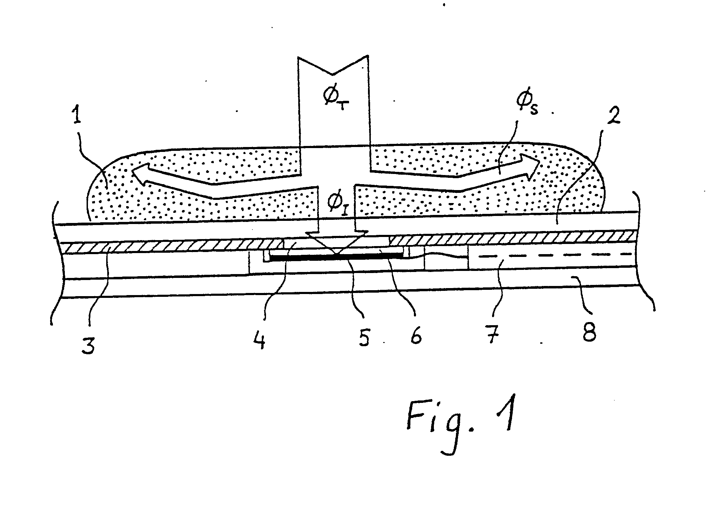

The apparatus described in this invention removes the problem which arises in the interpretation of the results of agglutination tests. The apparatus replaces the human eye in reading the results of the latex agglutination test. The major feature of the apparatus is a light indicator, on top of which the agglutination reaction is performed. The light indicator measures the amount of light passing through the liquid. After the occurrence of an agglutination reaction the size of the light scattering centres increases and their number decreases, as a result of which the amount of light passing through the liquid increases. In conjunction with the light indicator there may also be e.g. an area made of suitably coloured no nabsorbant material with the aid of which it is possible to inspect the agglutination visually as in traditional methods. The apparatus may include more than one photodiode or phototransistor. The light entering the light indicator 5 (Figure 1) can be filtered with the light filter 6. The amount $\varnothing_I$ of light entering the light indicator 5 is proportional to the difference between the amount $\varnothing_T$ of light entering the drop 1 from the environment and the amount $\varnothing_S$ of light leaving the drop after scattering, absorption, reflection and refraction. The amount of light scattered in the drop 1 is proportional to the size and the number of particles within the drop. The light indicator can therefore be used to observe changes occurring in the size and the number of particles in the drop. The light indicator 5 and the signal processing circuit 7 are protected by the plastic covering 8.

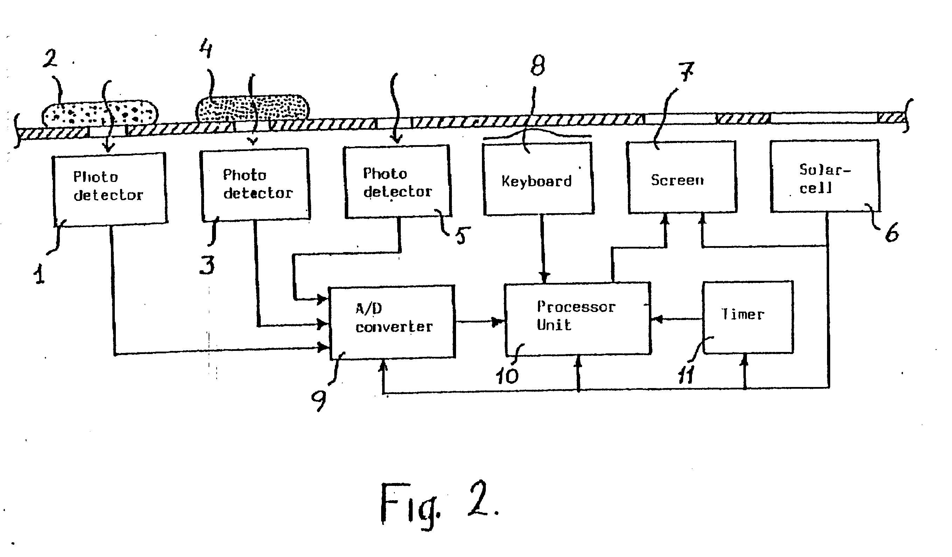

A flow chart of one apparatus for carrying out this type of method is presentd in Figure 2.

The apparatus may hold three light indicators with different functions .The light indicator 1 measures the light passing through the sample drop 2. The sample drop 2 contains the sample and the reagent. The light indicator 3 measures the light passing through the reference drop 4. This reference drop 4 may contain negative control reagents. The light indicator 5 measures the light intensity in the environment. When the apparatus is placed in a position with sufficient illumination the solar cell 6 provides electric current and the apparatus can be used for measurements. The electrical energy required for the functioning of the apparatus can also if necessary be obtained from an internal source such as a battery or accumulator. The readiness of the apparatus for use is indicated by the display 7. This display is also an indication of sufficient illumination for the carrying out of the test and of sufficient state of charge of the battery or accumulator if used. The test is started by pressing the zeroing button 8, upon which the display 7 is zeroed and the signal generated by each light indicator is tranformed to the digital mode in the A/D-converter 9 and memorized in the signal processing unit 10. At the same instant the timer 11 begins to operate. When the timer 11 has counted a preordained lenght of time T, the signals given by the light indicators are transferred again to the signal processing unit 10. On the basis of the two measurement results given by the light indicator 5, the change d $\varnothing_T$ occurring in the background illumination during the time interval T is calculated. Similarly, using the corresponding signals provided by the light indicators 1 and 3, the changes d $\varnothing_S$ and d $\varnothing_R$ in the light passing through the sample drop 2 and the reference drop 4 during the time interval T are calculated. The change d $\varnothing_S$ in the signal corresponding to the change in the light passing through the sample drop 2 is corrected for the corresponding changes d $\varnothing_T$ and d $\varnothing_R$ in the encironment and in the reference drop 4, respectively, scaled and taken to the display 7. If it is required only to indicate whether the change occurring in the sample drop 2 exceeds a certain threshold value, a comparator can be used in place of the A/D-converter, i.e. a single-bit A/D conversion is effected.

## Claims

1. An apparatus for interpreting the results of agglutination tests, in which the apparatus includes a light indicator with which it is possible to observe the change, resulting from an agglutination reaction, in the amount of light reaching the light indicator via the route taken by external illumination, not generatd by the apparatus itself, to the light indicator; and in the agglutination reaction which is carried out in the aforesaid route of light reaching the apparatus from an external source, not generated by the apparatus, a carrier is used which is coated with a biologically interesting material which can be identified by an agglutination reaction.

2. An apparatus according to claim 1 in which the carrier used in the agglutination reaction is a latex particle or a particle prepared from some other organic material an animal red blood cell, a particle made of inorganic material, a microbe or a liposome.

3. An apparatus according to claim 1 in which the light indicator is a light resistor, a photodiode or a phototransistor.

4. An apparatus according to claim 2 in which the carrier used in the agglutination reaction is a latex particle.

5. An apparatus according to claims 1-4 in which the power source of the apparatus is a solar cell.

6. A method for the carrying out of an agglutination test using an apparatus registering agglutination, in which an apparatus is used which includes a light indicator with which it is possible to observe a change, caused by an agglutination reaction, in the amount of light reaching the light indicator via the route taken by external illumination, not generated by the apparatus itself, to the light indicator; and in the agglutination reaction carried out in the aforesaid route of light reaching the apparatus from an external source, not generated by the apparatus, a carrier is used which is coated with a biologically interesting material which can be identified by an agglutination reaction.

7. A method according to claim 6 in which the carrier in the agglutination reaction is a latex particle or a particle prepared from some other organic material, an animal red blood cell, a particle made of inorganic material, a microbe or a liposome.

8. A method arrording to claim 6 in which the light indicator is a photoresistor, a photodiode or a phototransistor.

9. A method according to claim 7 in which the carrier particle used in the agglutination reaction is a latex particle.

10. A method according to claims 7-9 in which the power source of the apparatus is a solar cell.

Fig. 1

Fig. 2.

0 218 188

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 250 394 (J.J. O'CONNOR) <br><br> * Abstract; figure 1; column 5, line 57 - column 7, line 59; column 13, line 24 - column 14, line 66; column 16, lines 37-59 * | 1-4,6-9 | G 01 N 21/82 <br> G 01 N 33/546 |
| A | US-A-4 208 185 (Y. MASANOBU SAWAI et al.) <br> * Abstract; figure 4; column 1, lines 36-56; column 3, line 66 - column 4, line 63; column 8, lines 19-32; column 12, line 54 - column 13, line 68 * | 1-4,6-9 | |
| A | FR-A-2 509 861 (S. LOUIS et al.) <br> * Page 1, line 19 - page 2, line 5; page 3, lines 23-32; page 4, line 22 - page 5, line 22 * | 1-3,6-8 | **TECHNICAL FIELDS SEARCHED (Int. Cl.4)** <br><br> G 01 N |
| A | US-A-4 305 721 (D. BERNSTEIN) <br><br> * Abstract; column 1, lines 12-62; column 2, lines 44-59; column 3, lines 41-57 * | 1,2,4, 6,7,9 | |

-----

The present search report has been drawn up for all claims

| Place of search | Date of completion of the search | Examiner |
|---|---|---|
| THE HAGUE | 07-01-1987 | HITCHEN C.E. |